# EUROPEAN PATENT APPLICATION

(11) **EP 2 487 139 A1**
(43) Date of publication of application: **15.08.2012**
(21) Application number: 10821903.1
(22) Date of filing: 29.09.2010
(51) Int. Cl.: C02F 3/12, C02F 1/44, C02F 3/34, C12M 1/00, C12N 1/20

(54) **ADDITIVE USED IN A MEMBRANE-SEPARATION ACTIVATED SLUDGE PROCESS**

(30) Priority: 05.10.2009 JP 2009231554
(71) Applicant: Asahi Kasei Chemicals Corporation, Tokyo 101-8101 (JP); National University Corporation Nagoya Institute of Technology, Nagoya-shi, Aichi 466-8555 (JP)
(72) Inventor: OKAMURA, Daisuke, Tokyo 101-8101 (JP); HORI, Katsutoshi, Nagoya-shi Aichi 458-0015 (JP)
(74) Representative: Strehl Schübel-Hopf & Partner
(86) International application number: PCT/JP2010/066947
(87) International publication number: WO 2011/043232

(57) **Abstract**

In order to provide a means that can process organic wastewater via a membrane-separation activated sludge process that is stable over a long period of time, by reducing the quantity of biological polymers that can cause permeability problems, an additive is provided that is added to the activated sludge when treating organic wastewater via a membrane-separation activated sludge process. Said additive contains microorganisms that decompose polysaccharides.

## Description

### Technical Field

The present invention relates to an additive for attaining a stable treatment when organic wastewater is treated with a membrane separation activated sludge method.

### Background Art

As a wastewater-treating method, there is a membrane separation activated sludge method in which a membrane cartridge is immersed as a separation membrane device in an activated sludge tank and a solid-liquid separation between an activated sludge and a treated liquid is conducted by filtration. The method enables to conduct the solid-liquid separation under the conditions that the activated sludge (mixed liquor suspended solid: MLSS) concentration is set at an extremely high value of from 5000 to 20000 mg/L. Accordingly, the method offers an advantage such that the volume of the activated sludge tank can be reduced or the reaction time in the activated sludge tank can be reduced. Additionally, the method can reduce the site area for the treatment facilities because the method adopts the filtration with a membrane, hence no suspended solid (SS) is mixed in the treated water, and consequently no final sedimentation tank is needed. Furthermore, the method permits the filtration irrespective of the sedimentation ability of the activated sludge, and hence reduces the burden on activated sludge control. Recently, the membrane separation activated sludge method that has many merits as described above become widely used.

For a membrane cartridge, a flat membrane or a hollow fiber membrane is used. Use of a hollow fiber membrane offers a high strength of the membrane itself, and hence alleviates the damage of the membrane surface due to the contact with the contaminants originating from the organic wastewater, and thus the membrane cartridge using a hollow fiber membrane is durable to a relatively long term use. Further, use of a hollow fiber membrane also offers an advantage such that backwash can be conducted to remove the adhesion substances on the membrane surface by spouting a medium such as filtration water in a direction opposite to the filtration direction. However, because the effective membrane area is reduced by adhesion, on the membrane surface, of biological polymers as metabolized by the microorganisms in the activated sludge and/or by adhesion, on the membrane surface, of the activated sludge so as to degrade the filtration efficiency, the period during which filtration can be stably conducted is limited.

To solve such problems as described above, for example, Japanese Patent Laid-Open No. 2000-157846 (Patent Literature 1) discloses a method in which by aerating with air or the like from the lower portion of the membrane cartridge, the activated sludge aggregates and/or the contaminants brought in by the raw water on the surface of the hollow fiber membranes and between the hollow fiber membranes are detached through the effect of the membrane oscillation and the stirring effect of the upward movement of the gas bubbles so as to prevent the accumulation of the sludge aggregates and/or the contaminants. According to the method, for example, a lower ring is disposed at a lower portion of the hollow fiber membrane cartridge, a plurality of through-holes are arranged on an adhesion fixing layer disposed on the lower ring, an air reservoir is formed in the lower ring by aeration from the cartridge lower portion, thus air bubbles are uniformly generated from the plurality of through-holes and consequently the activated sludge, the contaminants and the like attached to the outer surface of the hollow fiber membranes are detached.

On the other hand, Japanese Patent Laid-Open No. 2005-40747 (Patent Literature 2) discloses a method in which the amount of the biological polymers in the activated sludge mixed solution is measured, and thus the amount of the biological polymers in an biological treatment tank (aeration tank) is timely reduced to prevent the adhesion of the excessive polymers on the surface of the membranes.

Additionally, Japanese Patent Laid-Open No. 2007-260664 (Patent Literature 3) discloses a technique for preventing the membrane clogging by adding, to the activated sludge, animalcules that prey on prokaryotic organisms responsible for causing the membrane clogging.

### Prior Art Documents

### Patent Literature

[PTL 1] Japanese Patent Laid-Open No. 2000-157846
[PTL 2] Japanese Patent Laid-Open No. 2005-40747
[PTL 3] Japanese Patent Laid-Open No. 2007-260664

### Summary of Invention

### Technical Problem

However, in the method described in Patent Literature 1, when the organic matter concentration in the organic wastewater is varied widely, or when the oxidants, acidic liquid, basic liquid and the like flow in the activated sludge tank, microorganisms may discharge to the outside thereof metabolites (referred to as biological polymers) in extraordinary amounts. Under the continuation of the conditions in which concentration of the biological polymers is extraordinarily high, the biological polymers attached to the outer surface of the membranes can no longer be detached sufficiently by aeration, and the membrane filtration resistance will increase. Additionally, in the method described in Patent Literature 2, the chemical oxygen demand (COD) value is obtained to be used as a substitute for the amount of the biological polymers. There arise problems such that the COD detects even the organic matter that can pass, without stopping, through the pores of the membranes. Further, the method described in Patent Literature 3 is unable to cope with the clogging due to the biological polymers.

With the above-described background, the present invention relates to the membrane separation activated sludge method and takes as its object the provision of a procedure capable of reducing the amount of the biological polymers responsible for causing the permeability failure and capable of stably attaining over a long period of time the treatment of organic wastewater based on the membrane separation activated sludge method.

### Solution to Problem

The present inventors made a diligent study for the purpose of achieving the above-described object, and consequently achieved the present invention by discovering that the treatment of organic wastewater can be stably continued over a long period of time by adding, to an activated sludge, an additive that contains a microorganism to degrade a polysaccharide responsible for causing the membrane clogging and by discovering an inexpensive and efficient method for preparing such an additive.

Specifically, the present invention relates to an additive which is added to an activated sludge when organic wastewater is treated with a membrane separation activated sludge method, the additive comprising: a microorganism to degrade a polysaccharide.
The present invention also relates to said additive, wherein the polysaccharide is a polysaccharide comprising an uronic acid unit.
The present invention also relates to said additive, wherein the polysaccharide is a polysaccharide comprising the uronic acid unit in a content of 5 to 70% based on total saccharide unit.
The present invention also relates to said additive, wherein the polysaccharide is one or more polysaccharides selected from polygalacturonic acid, xanthan gum and hyaluronic acid.
The present invention also relates to said additive, wherein the microorganism is one or more microorganisms selected from Penicillium sp., Phialemonium sp., Fluviicola sp., Pedobacter sp., Paenibacillus sp., Cohnella sp., Pseudoxanthomonas sp., Brevundimonas sp., Hydrogenophaga sp., Sphingomonas sp., Novosphingobium sp., Sphingopyxis sp., Microbacterium sp. Ochrobactrum sp., Sphingobacterium sp., Bacteroidetes bacterium, Xanthomonadaceae sp., Devosia sp., Prosthecomicrobium sp., Alpha proteobacterium sp. and Flexibacteraceae sp..
The present invention also relates to said method, wherein the uronic acid unit-containing polysaccharide is one or more polysaccharides selected from polygalacturonic acid, xanthan gum and hyaluronic acid. Further, the present invention relates to an organic wastewater-treating method based on a membrane separation activated sludge method comprising:
an inflow step in which an organic wastewater is made to flow in an activated sludge tank that contains a microorganism-containing activated sludge; and
a separation step in which a treated liquid obtained by biologically treating the organic wastewater in the activated sludge tank is subjected to a solid-liquid separation with a separation membrane device disposed in the activated sludge tank,
wherein an additive which contains a microorganism to degrade an uronic acid unit-containing polysaccharide is added to the activated sludge.
The present invention also relates to said organic wastewater-treating method, wherein the uronic acid unit concentration change over time in the water phase in the activated sludge tank is being measured, and the additive is added when the uronic acid unit concentration reaches 30 mg/L. Further, the present invention relates to an organic wastewater-treating apparatus based on a membrane separation activated sludge method, comprising:
an activated sludge tank in which a microorganism-containing activated sludge is contained, and an organic wastewater is subjected to a biological treatment;
a separation membrane device disposed in the activated sludge tank or at a stage subsequent to the activated sludge tank and subjecting the biologically treated water to a solid-liquid separation;
a concentration-measuring means for measuring an uronic acid unit concentration change over time in the water phase in the activated sludge tank; and
an additive-adding means for adding in the activated sludge an additive that contains a microorganism to degrade an uronic acid unit-containing polysaccharide when the uronic acid unit concentration reaches a predetermined concentration.

### Advantageous Effects of Invention

By adding the additive according to the present invention to the activated sludge, a polysaccharide is degraded which is a substance responsible for causing the membrane clogging in the membrane separation activated sludge method, and consequently the treatment of organic wastewater based on the membrane separation activated sludge method can be conducted stably over a long period of time. Additionally, the additive according to the present invention can be prepared by using an activated sludge, soil, lake water, river water and the like with an inexpensive, simple and easy method.

### Brief description of Drawings

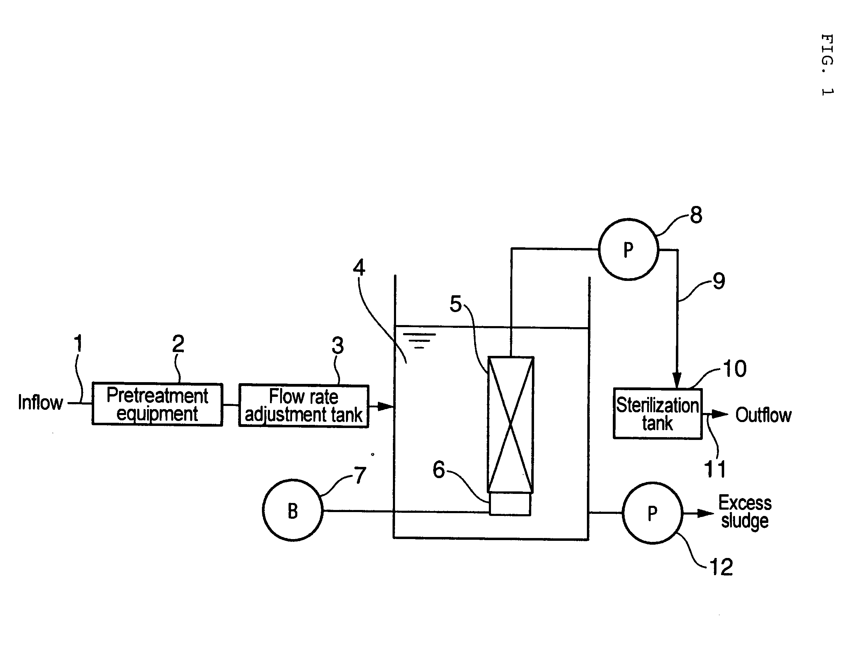

FIG. 1 shows a block diagram illustrating an organic wastewater-treating method using a membrane separation activated sludge method.

### Description of Embodiments

Hereinafter, an embodiment (hereinafter, referred to as "the present embodiment") for carrying out the present invention is described in detail. It is to be noted that the present invention is not limited to the present embodiment described below, and may be implemented in various modifications within the scope of the gist thereof. Additionally, in the accompanying drawing, the relative positions reflect the actual relative positions unless otherwise specified, but the dimensional proportions do not necessarily represent the actual dimensional proportions.

### (Membrane separation activated sludge method)

First, described is the membrane separation activated sludge method in which the additive according to the present embodiment is used.

The membrane separation activated sludge method includes: an inflow step in which an organic wastewater is made to flow in an activated sludge tank that contains a microorganism-containing activated sludge; and a separation step in which a treated liquid obtained by biologically treating the organic wastewater in the activated sludge tank is subjected to a solid-liquid separation with a separation membrane device disposed in the activated sludge tank.

In the inflow step, first, pretreatment equipment removes contaminants from the organic wastewater in such a rough way that large-sized solid contents and the like are removed. The organic wastewater thus pretreated is once stored in a flow rate adjustment tank, and then transferred into an activated sludge tank while the flow rate thereof is being adjusted.

In the successive separation step, in the activated sludge tank, first, microorganisms in the activated sludge degrade the organic matter in the organic wastewater (such a biodegradable organic matter is also referred to as BOD component). The size of the activated sludge tank and the retention time of the organic wastewater in the activated sludge tank can be appropriately determined according to the treatment amount of the organic wastewater in the activated sludge tank and the organic matter concentration in the organic wastewater. In general, the activated sludge concentration in the activated sludge tank is preferably about 5 to 20 g/L, but is not limited to this range.

In the separation step, next, a separation membrane device conducts a solid-liquid separation between the activated sludge and the organic wastewater in the activated sludge tank. The immerse-type separation membrane device disposed in the activated sludge tank includes separation membranes and water-collecting sections. The water-collecting sections in the separation membrane device are connected through pipes to a suction pump, the suction pump generates a pressure gradient between the inner surface and the outer surface of each of the membranes to achieve the solid-liquid separation.

For the membrane cartridge used as the separation membranes, heretofore known separation membranes such as flat membranes and hollow fiber membranes can be used. Among these membranes, hollow fiber membranes are high in the strengths of the membranes themselves, hence alleviate the damage to the surface of the membranes due to the contact with the contaminants in the organic wastewater, and are durable to a relatively long term use.

The pore size and the material of the filtration membrane is not particularly limited as long as the filtration is satisfactorily conducted; for example, a filtration membrane made of polyvinylidene difluoride (PVDF) having a pore size of about 0.1 µm is preferably used.

For the purpose of preventing the filtration membrane clogging, as a physical technique, a skirt is disposed in the separation membrane device and gas may be fed from a blower into the skirt to oscillate the membranes, or water flow is applied to the membrane surface to exert shear stress to the membranes. Alternatively, by spouting filtration water or the like in a direction opposite to the filtration direction, backwashing may also be conducted so as to remove the adhesion substances on the surface of the membranes.

The separation membrane device may be disposed in the activated sludge tank as immersed therein or alternatively at a stage subsequent to the activated sludge tank as connected thereto. Accordingly, the additive according to the present embodiment is applicable not only to the membrane separation activated sludge method using an immersion-type separation membrane device but also to the cases such as a case where a separation membrane device is disposed in a tank other than the activated sludge tank and a case where a pressurized separation membrane device is used. In such methods, the activated sludge is circulated between the activated sludge tank and the separation membrane device so as to return the concentrated liquid to the activated sludge tank.

The separation membranes may be disposed as a plurality of series where necessary. The adoption of the plurality of series permits independent operation of the series in such a way that in each of the series of the separation membranes, the separation operation is conducted or ceased, and hence permits the adjustment of the wastewater treatment speed and/or the maintenance management of the separation membranes.

Organic wastewaters that can be treated with the membrane separation activated sludge method using the additive of the present embodiment are not particularly limited; examples of such organic wastewaters include food factory wastewater, sugar factory wastewater, detergent factory wastewater, starch factory wastewater and bean curd factory wastewater.

### (Wastewater-treating apparatus)

The exemplary apparatus used for the wastewater-treating method based on the above-described membrane separation activated sludge method is an apparatus illustrated in FIG. 1.

First, the organic wastewater 1 is subjected to removal of the contaminants therefrom in the pretreatment equipment 2, thereafter once stored in the flow rate adjustment tank 3, and then fed from the flow rate adjustment tank 3 to the activated sludge tank (aeration tank) 4 at a constant flow rate.

In the activated sludge tank 4, the microorganisms in the activated sludge placed in the tank degrade and remove the organic matter (BOD component) in the organic wastewater 1. The solid-liquid separation of the activated sludge mixed liquid in the activated sludge tank 4 is conducted with the separation membrane device 5 immersed in the tank. In the lower portion of the separation membrane device 5, a skirt 6 and a blower 7 are disposed, and gas is fed from the blower 7 into the skirt 6. The filtrate 9 having been treated with the separation membrane device 5 is sucked with a suction pump 8, sterilized where necessary in a sterilization tank 10, and then discharged as outflow of treated water 11. The excess sludge is, where necessary, withdrawn from the activated sludge tank (Aeration tank) 4 with a sludge withdrawal pump 12.

### (Additive)

Next, the additive according to the present embodiment is described. The additive according to the present embodiment is added to the activated sludge in the above-described membrane separation activated sludge method.

In the activated sludge tank, microorganisms degrade the organic matter and also discharge to the outside thereof metabolites. In a case where organic matter flows excessively in the activated sludge tank, in a case where the organic matter concentration in the influent water is varied widely, or in a case where an oxidant, an acidic liquid, a basic liquid or the like flows in the activated sludge tank the metabolites of the microorganisms are remarkably discharged to the outside thereof to promote the separation membrane clogging. The present inventors have revealed that the metabolites are polysaccharides, in particular, uronic acid unit-containing polysaccharides.

The additive according to the present embodiment contains microorganisms capable of degrading such polysaccharides, and the addition of the additive enables to degrade the polysaccharides metabolized by the microorganisms in the activated sludge so as to prevent the separation membrane clogging. Examples of the polysaccharides degraded by the microorganisms contained in the additive according to the present embodiment include neutral polysaccharides such as starch, aminopolysaccharides such as chitin, acidic polysaccharides such as polyuronic acid and uronic acid unit-containing polysaccharides such as xanthan gum. Preferable among these are the uronic acid unit-containing polysaccharides.

The uronic acid unit-containing polysaccharides are not particularly limited as long as the uronic acid unit-containing polysaccharides contain as the constituent unit thereof the uronic acid that is a carboxylic acid obtained by converting the hydroxymethyl group (-CH₂OH) at the main chain terminal into a carboxyl group (-CO₂H), among the derivatives obtained by oxidizing monosaccharide; examples of the uronic acid unit-containing polysaccharides include, but are not limited to: xanthan gum, hyaluronic acid, alginic acid, heparin, and chondroitin sulfate which are polysaccharides containing as the constituent units thereof uronic acid unit-containing monosaccharides such as glucuronic acid, galacturonic acid, mannuronic acid and iduronic aid; and polygalacturonic acid as a polyuronic acid that is a polymer formed only of the uronic acid unit. Preferable among these, as the uronic acid unit-containing polysaccharide, is xanthan gum, polygalacturonic acid or hyaluronc acid, more preferable is xanthan gum or polygalacturonic acid, and particularly preferable is xanthan gum. The microorganisms contained in the additive according to the present embodiment may also be microorganisms capable of degrading one or more of these polysaccharides.

The uronic acid unit-containing polysaccharide is preferably a polysaccharide that contains the uronic acid unit and other saccharide units, from the viewpoint of enhancing the efficiency of the degradation of the clogging substances. For example, the uronic acid unit concentration is preferably 5 to 70%, more preferably 7 to 60% and particularly preferably 10 to 50% of the concentration of the total saccharide unit. In this connection, the concentration of the total saccharide unit can be measured by the below-described phenol-sulfuric acid method.
1) In a test tube, 500 µL of a sample aqueous solution or a standard monosaccharide aqueous solution is placed.
2) To the test tube, 500 µL of a 5 wt% phenol aqueous solution is added and stirred.
3) To the test tube, 2.5 mL of concentrated sulfuric acid is added, and immediately, vigorously stirred for 10 seconds.
4) The test tube is allowed to stand in a water bath set at 30°C for 20 minutes or more.
5) The light absorption at 490 nm is measured on a spectrophotometer, to obtain the concentration from the calibration curve prepared with the standard monosaccharide aqueous solutions having known concentrations.
It is to be noted that the uronic acid unit concentration can be measured by using the below-described technique.

Additionally, the microorganisms contained in the additive of the present embodiment are not particularly limited as long as the microorganisms degrade the above-described polysaccharides; one type of microorganism or a mixture of two or more types of microorganisms may be used, and the additive of the present embodiment containing the microorganisms can be obtained by, for example, the below-described method.

### (Method of preparing the additive)

The exemplary method of preparing the additive according to the present embodiment is a method including: a first step in which to a culture medium with carbon source consisting only of polysaccharide(s), preferably uronic acid unit-containing polysaccharide(s), at least one member selected from the group consisting of an activated sludge, soil, lake water and river water is added and cultured, and microorganism(s) are harvested from the colonies having a degradation ability for the uronic acid-containing polysaccharide(s); and a second step of preparing the additive that contains the microorganism(s).

As the culture medium of the first step, for example, an agar medium containing, in addition to the polysaccharide as the carbon source, nutrient salts of nitrogen and phosphorus, and trace of metal salts may be used. On the surface of an agar flat plate, an activated sludge, soil, lake water, river water or the like is uniformly inoculated, and then screening is conducted. The microorganisms that proliferate on such a plate are the microorganisms capable of degrading polysaccharide, and hence by harvesting such proliferating microorganisms, polysaccharide-degrading microorganisms can be obtained.

The harvesting of the microorganisms can be conducted by fishing with a platinum loop from the colonies degrading the polysaccharide on the agar culture medium. When a suspension culture medium containing polysaccharide is obtained, if the polysaccharide is degraded and assimilated by the microorganisms, the formation of transparent degradation spots can be observed. Accordingly, the colonies having degradation ability for the polysaccharide can be easily recognized. For example, a powder of polygalacturonic acid is used as the polysaccharide, an opaque suspension culture medium is obtained; thus, when the polygalacturonic acid is degraded and assimilated, the formation of transparent degradation spots (also referred to as halo) can be observed. Such colonies contain the polysaccharide-degrading microorganisms, and such microorganisms may be of one type or mixtures of two or more types.

Next, in the second step, the one type of microorganism thus obtained or the mixture of two or more types of microorganisms thus obtained is converted into a form of additive for the purpose of facilitating the addition to the activated sludge. For example, the culture solution of the polysaccharide-degrading microorganisms, obtained in the first step, may be diluted with sterile saline or the like to yield a liquid additive, or may be concentrated based on a technique such as gravitational-centrifugal separation, membrane separation or stationary sedimentation separation to yield an additive. The liquid additive may be supported by a carrier such as a sponge. Alternatively, the culture solution may be subjected to freeze-dry treatment to yield an additive, or after the freeze-drying of the culture solution, the thus obtained additive may be converted into a powder or molded into a pellet.

The polysaccharide-degrading microorganisms are not particularly limited as long as the polysaccharide-degrading microorganisms each have an desired degradation ability for polysaccharides, preferably, the degradation ability for uronic acid unit-containing polysaccharides; one type of microorganism or a mixture of two or more types of microorganisms which are each verified to have such a degradation ability by the above-described techniques may be appropriately used. Examples of such microorganisms include one or more microorganisms selected, for example, from Penicillium sp., Phialemonium sp., Fluviicola sp., Pedobacter sp., Paenibacillus sp., Cohnella sp., Pseudoxanthomonas sp., Brevundimonas sp., Hydrogenophaga sp., Sphingomonas sp., Novosphingobium sp., Sphingopyxis sp., Microbacterium sp. Ochrobactrum sp., Sphingobacterium sp., Bacteroidetes bacterium, Xanthomonadaceae sp., Devosia sp., Prosthecomicrobium sp., Alpha proteobacterium sp. and Flexibacteraceae sp.. From the viewpoint of greater degradation ability for uronic acid unit-containing polysaccharides, preferably, such microorganisms include one or more microorganisms selected from Fluviicola sp., Pedobacter sp., Paenibacillus sp., Cohnella sp., Pseudoxanthomonas sp., Brevundimonas sp., Hydrogenophaga sp., Sphingomonas sp., Novosphingobium sp., Sphingopyxis sp., Microbacterium sp. Ochrobactrum sp., Sphingobacterium sp., Bacteroidetes bacterium, Xanthomonadaceae sp., Devosia sp., Prosthecomicrobium sp., Alpha proteobacterium sp. and Flexibacteraceae sp.. The microorganisms can be identified by using a technique well known to those skilled in the art, with reference to the following Examples, etc.

The additive may appropriately formulated according to the types of the polysaccharide-degrading microorganisms, and the content of the organic solid substances and other properties of the activated sludge to which the additive is to be added. Usually, when the number of bacteria in the activated sludge is about 10² to 10⁵ CFU/mL, the uronic acid unit-containing polysaccharide can be rapidly degraded. Accordingly, the formulation is favorably conducted with such a concentration that, for example, when the additive is transplanted in the activated sludge in a content of 1% [V/V], the number of bacteria in the additive contained in the sludge is about 10² to 10⁵ CFU/mL.

### (Wastewater-treating method)

The wastewater-treating method according to the present embodiment is characterized in that the additive is added when the uronic acid unit concentration reaches a predetermined concentration, for example, 30 mg/L, while the uronic acid unit concentration change over time in the water phase of the activated sludge tank is being measured.

As described above, the polysaccharides metabolized by the microorganisms in the activated sludge contributes to the clogging of the separation membrane in the membrane separation activated sludge method. Accordingly, the measurement of the saccharide concentration, preferably, the uronic acid unit concentration in the water phase of the activated sludge contained in the activated sludge tank enables to appropriately evaluate the risk of clogging of the separation membranes by the uronic acid unit-containing polysaccharides, and by adding the additive in appropriate time and in appropriate amount based on this evaluation, the membrane separation can be stably and efficiently attained.

In general, the uronic acid unit concentration in the water phase of the activated sludge is preferably 50 mg/L or less, more preferably 30 mg/L or less, furthermore preferably 20 mg/L or less and most preferably 10 mg/L or less. Accordingly, the additive according to the present embodiment is preferably added so as to maintain the uronic acid unit concentration to the concentration equal to or less than these concentrations; however, the membrane clogging hardly occurs until the uronic acid unit concentration reaches 30 mg/L, and hence it is economically advantageous to add the additive when the uronic acid unit concentration reaches 30 mg/L. On the other hand, when the uronic acid unit concentration reaches 50 mg/L, the membrane clogging starts to occur, and hence it is preferable to add the additive before the uronic acid unit concentration reaches 50 mg/L, preferably, 40 mg/L.

The measurement of the uronic acid unit concentration in the water phase of the activated sludge is preferably conducted with the sludge filtrate having been obtained by filtering the activated sludge with a filter medium such as a filter paper having a pore size larger than the pore size of the separation membranes in the separation membrane device. By such filtration operation, only the suspended solid in the activated sludge is captured by the filter medium and the saccharide component passes through the filter paper. Consequently, the measurement of the total saccharide unit concentration and/or the uronic acid unit concentration in the filtrate enables to know more accurately the concentration of the polysaccharides of biological origin which are substances responsible for causing the membrane clogging.

The pore size of the filter medium is preferably five or more times and more preferably ten or more times the pore size of the separation membranes disposed in the separation membrane device. The upper limit of the pore size of the filter medium is preferably about one hundred or less times the pore size of the separation membranes disposed in the separation membrane device, and is more preferably set at 10 µm. Further, the material of the filter medium is preferably hydrophilic because of the smaller adsorption of the saccharide component, and for example, a filter paper made of a material such as cellulose may be used.

It is to be noted that the uronic acid unit concentration as referred to in the present embodiment means the concentration of only the uronic acid unit in the uronic acid unit-containing polysaccharides. According to the following method described in "New Method for Quantitative Determination of Uronic Acid," by NELLY BLUMENKRANTZ and GUSTAV ASBOE-HANSEN, in ANALYTICAL BIOCHEMISTRY, Vol. 54, pp. 484 to 489 (published in 1973), the uronic acid concentration can be measured with a calibration curve prepared by using polygalacturonic acid that is a polyuronic acid.
1) Each of 0.5 mL of the sludge filtrate and a polygalacturonic acid aqueous solution of a known concentration is placed in a test tube, and to each of the test tubes, 3.0 mL of a 0.0125 M Na₂B₄O₇ concentrated sulfuric acid solution is added.
2) Each of the solutions thus obtained is shaken sufficiently, then heated in a boiling water bath for 5 minutes and thereafter cooled in an ice water bath for 20 minutes.
3) To each of the solutions, 50 µL of a 0.15% m-hydroxydiphenyl solution in 0.5% NaOH solution is added.
4) Each of the solutions thus obtained is shaken sufficiently, and 5 minutes later, each of the solutions prepared in 3), absorbance at 520 nm is measured, to obtain the uronic acid concentration in the sludge filtrate from a comparison between the value of the polygalacturonic acid aqueous solution of a known concentration and the value of the sludge filtrate.

In one aspect, the present embodiment provides an organic wastewater-treating apparatus (this apparatus may be an existing apparatus) based on a membrane separation activated sludge method, the apparatus including: a concentration-measuring means for measuring the uronic acid unit concentration change over time in the water phase in the activated sludge tank; and an additive-adding means for adding the additive when the uronic acid unit concentration reaches a predetermined concentration. According to the apparatus, the addition of the additive is conducted based on the uronic acid unit concentration, and hence the apparatus enables to conduct a continuous wastewater treatment economically. The measurement of the uronic acid unit concentration can be conducted, for example, with reference to the above-presented description. Additionally, the additive-adding means can be implemented by using a technique well known to those skilled in the art. Also, the addition of the additive can be conducted automatically.

### Examples

Hereinafter, the present embodiment is described more specifically with reference to Examples and Comparative Examples. However, the scope of the present invention is not limited to these Examples. In the Examples and Comparative Examples, the microorganisms were identified by 28S or 16S rRNA gene analysis.

### Example 1

An agar flat plate culture medium containing 0.15% of a phosphorus content, 0.04% of a nitrogen content and 0.001% of a Mg content was prepared. Polygalacturonic acid (Sigma Aldrich Corp.) was dispersed in an agar solution and the dispersed solution was laminated on the culture medium. Thus, an agar flat plate culture medium containing polygalacturonic acid as an only carbon source was prepared.

To the culture medium, an activated sludge, soil, and lake water were uniformly inoculated, and the culture medium was incubated at 28°C for 72 hours to form colonies of molds on the agar flat plate. The colony contained at least molds such as Penicillium sp. and Phialemonium sp.

The molds grown on the agar flat plate were aseptically inoculated with a platinum pool in 20 mL of a liquid culture medium that contains 0.15% of a phosphorus content, 0.04% of a nitrogen content and 0.001% of a Mg content as nutrient salts and further contains polygalacturonic acid in a concentration of 1 g/L as an only carbon source.

The liquid culture medium was cultured for 24 hours while being maintained at 28°C, shaken at 120 rpm and aerated. 24 hours later, the uronic acid unit concentration was measured and found to be decreased to a concentration of 0.7 g/L. Hereinafter, the culture solution thus obtained is referred to as the culture solution A (additive).

Subsequently, the activated sludge in which the uronic acid unit concentration determined by the above-described method reached 100 mg/L was filtered with a filter paper 5C manufactured by Advantec MFS, Inc. to prepare a filtrate. 1 L of the filtrate was placed in a 5-L Erlenmeyer flask, 10 mL of the above-described culture solution A was aseptically added to the culture solution, and the thus obtained solution was subjected to a shaking and aeration treatment for 24 hours at a rate of 120 rpm while being maintained at 28°C.

24 hours later, the uronic acid unit concentration was measured with the above-described method, and was verified to be decreased to 50 mg/L. The molecular weight distribution of the concerned solution was measured by applying the following conditions to verify that the peaks indicating the molecular weights of 1×10⁶ to 1×10⁸ Da became smaller in height.

The conditions: For the measurement of the molecular weight distribution, HPLC system D7000 series manufactured by Hitachi Co., Ltd. was used, and the column used was a size-exclusion column (Shodex-OH pack SB-806HQ, Showa Denko K.K.). The column temperature was set at 40°C. For mobile phase, a 0.05 M KH₂PO₄ buffer (pH 3.0) was delivered at a flow rate of 1.0 mL/min. 200 µL of a sample was injected, and the detection was made with a refractometer. With pullulans respectively having molecular weights of 1× 10⁴, 1× 10⁵, 1× 10⁶, 1× 10⁷, 1× 10⁸ and 1× 10⁹ Da, a calibration curve was drawn, and the molecular weight of the sample was determined by using the calibration curve.

### Example 2

An agar flat plate culture medium containing 0.15% of a phosphorus content, 0.04% of a nitrogen content, 0.001% of a Mg content and 1g/L of xanthan gum (Sigma Aldrich Corp.) was prepared. Thus, an agar flat plate culture medium containing xanthan gum as an only carbon source was prepared.

To the culture medium, an activated sludge, soil, and lake water were uniformly inoculated, and the culture medium was incubated at 28°C for 72 hours to form colonies of bacteria on the agar flat plate. The colony contained at least Microbacterium sp., Ochrobactrum sp., Pseudoxanthomonas sp., Fluviicola sp., Pedobacter sp., Paenibacillus sp., Cohnella sp., Brevundimonas sp., Hydrogenophaga sp., Sphingomonas sp., Novosphingobium sp. Sphingopyxis sp., Sphingobacterium sp., Bacteroidetes bacterium, Xanthomonadaceae sp., Devosia sp., Prosthecomicrobium sp., Alpha proteobacterium sp. and Flexibacteraceae sp..

The colony of bacteria grown on the agar flat plate were aseptically inoculated with a platinum pool in 20 mL of a liquid culture medium that contains 0.15% of a phosphorus content, 0.04% of a nitrogen content and 0.001% of a Mg content as nutrient salts and further contains xanthan gum (Sigma Aldrich Corp.) in a concentration of 1 g/L as an only carbon source.

The liquid culture medium was cultured for 24 hours while being maintained at 28°C, shaken at 120 rpm and aerated. 24 hours later, the uronic acid unit concentration was measured and found to be decreased to a concentration of 0.6 g/L. Hereinafter, the culture solution thus obtained is referred to as the culture solution B (additive).

Subsequently, the activated sludge in which the uronic acid unit concentration determined by the above-described method reached 100 mg/L was filtered with a filter paper 5C manufactured by Advantec MFS, Inc. to prepare a filtrate. 1 L of the filtrate was placed in a 5-L Erlenmeyer flask, 10 mL of the above-described culture solution B was aseptically added to the culture solution, and the thus obtained solution was subjected to a shaking and aeration treatment for 24 hours at a rate of 120 rpm while being maintained at 28°C.

24 hours later, the uronic acid unit concentration was measured with the above-described method, and was verified to be decreased to 40 mg/L, and the molecular weight distribution of the concerned solution was measured by applying a technique based on the same conditions as the conditions in Example 1 to verify that the peaks indicating the molecular weights of 1×10⁶ to 1×10⁸ Da became smaller in height.

### Example 3

An agar flat plate culture medium containing 0.15% of a phosphorus content, 0.04% of a nitrogen content, 0.001% of a Mg content and 1g/L of hyaluronic acid (Sigma Aldrich Corp.) was prepared. Thus, an agar flat plate culture medium containing hyaluronic acid as an only carbon source was prepared.

To the culture medium, an activated sludge, soil, and lake water were uniformly inoculated, and the culture medium was incubated at 28°C for 72 hours to form colonies of bacteria on the agar flat plate. The colony contained at least Fluviicola sp., Pedobacter sp., Paenibacillus sp., Cohnella sp., Brevundimonas sp., Hydrogenophaga sp., Sphingomonas sp., Microbacterium sp. and Ochrobactrum sp..

The colony of bacteria grown on the agar flat plate were aseptically inoculated with a platinum pool in 20 mL of a liquid culture medium that contains 0.15% of a phosphorus content, 0.04% of a nitrogen content and 0.001% of a Mg content as nutrient salts and further contains hyaluronic acid in a concentration of 1 g/L as an only carbon source.

The liquid culture medium was cultured for 24 hours while being maintained at 28°C, shaken at 120 rpm and aerated. 24 hours later, the uronic acid unit concentration was measured and found to be decreased to a concentration of 0.6 g/L. Hereinafter, the culture solution thus obtained is referred to as the culture solution C (additive).

Subsequently, the activated sludge in which the uronic acid unit concentration determined by the above-described method reached 100 mg/L was filtered with a filter paper 5C manufactured by Advantec MFS, Inc. to prepare a filtrate. 1 L of the filtrate was placed in a 5-L Erlenmeyer flask, 10 mL of the above-described culture solution C was aseptically added to the culture solution, and the thus obtained solution was subjected to a shaking and aeration treatment for 24 hours at a rate of 120 rpm while being maintained at 28°C.

24 hours later, the uronic acid unit concentration was measured with the above-described method, and was verified to be decreased to 49 mg/L, and the molecular weight distribution of the concerned solution was measured by applying a technique based on the same conditions as the conditions in Example 1 to verify that the peaks indicating the molecular weights of 1×10⁶ to 1×10⁸ Da became smaller in height.

### Example 4

By using the system illustrated in FIG. 1, a sugar factory wastewater having a BOD of 750 mg/L was treated with the membrane separation activated sludge method. The uronic acid unit concentration in the wastewater was 0 mg/L.

As a separation membrane device 5, a separation membrane device (Asahi Kasei Chemicals Corp., made of polyvinylidene difluoride (PVDF), membrane area: 0.015 m², effective membrane length: 15 cm, inner diameter/outer diameter: 0.6/1.2 mm) which is a module formed of precise filtration hollow fiber membranes of 0.1 µm in pore size was immersed in an activated sludge tank 4 having an effective volume of 10 L, and then suction filtration was conducted.

The MLSS concentration in the activated sludge tank was a constant value of 10 g/L, the retention time of wastewater in the activated sludge tank was set at 18 hours. The filtration pressure at the beginning of the treatment was 4 kPa. The liquid amount of the activated sludge was maintained at a constant value, the filtration flux was set at 0.6 m/D, and the total amount of the filtrate was discharged to the outside of the activated sludge tank. For aeration of the membranes, air was supplied from the lower portion of the membrane module at a flow rate of 200 L/h. Four such devices A, B, C and D were prepared and were made to start the operation under the same conditions.

The uronic acid unit concentration was obtained according to the same procedures as described above from the calibration curve for polygalacturonic acid. The uronic acid unit concentration in the water phase of the activated sludge was measured once a day.

About one week later from the start of the operation, the uronic acid unit concentration in the water phase of the activated sludge was increased rapidly; on the 11th day of the operation, the uronic acid unit concentration in the water phases of the activated sludges of the devices A, B, C and D was 50 mg/L, 55 mg/L, 53 mg/L and 50 mg/L, respectively. Then, the culture solutions A, B and C obtained respectively in Examples 1, 2 and 3 were added to the devices A, B and C, respectively, in an amount of 10 mL a day. On the 20th day from the start of the operation, the uronic acid unit concentrations in the water phases of the activated sludges of the devices A, B and C were decreased to 20 mg/L, 15 mg/L and 19 mg/L, respectively. The transmembrane pressure difference was not steeply increased in any of the devices and the devices were stably operated.

On the other hand, in the device D, 20 days after the start of the operation, the uronic acid unit concentration reached 60 mg/L, but the operation was continued as it was. Consequently, five days later, the filtration pressure exceeded 25 kPa, and thus the cleaning of the separation membranes was required.

### Comparative Example 1

An agar flat plate culture medium containing 0.15% of a phosphorus content, 0.04% of a nitrogen content, 0.001% of a Mg content and 1g/L of glucose was prepared. Thus, an agar flat plate culture medium containing glucose as an only carbon source was prepared.

To the culture medium, an activated sludge, soil, and lake water were uniformly inoculated, and the culture medium was incubated at 28°C for 72 hours to form colonies of bacteria on the agar flat plate. The colony contained at least Niabella sp., Terrimonas sp., Escherichia sp., Corynebacterium sp., Flavobacterium sp. and Opitutus sp..

The colony of bacteria grown on the agar flat plate were aseptically inoculated with a platinum pool in 20 mL of a liquid culture medium that contains 0.15% of a phosphorus content, 0.04% of a nitrogen content and 0.001% of a Mg content as nutrient salts and further contains glucose in a concentration of 1 g/L as an only carbon source.

The liquid culture medium was cultured for 24 hours while being maintained at 28°C, shaken at 120 rpm and aerated. 24 hours later, the total saccharide concentration was measured and found to be decreased to a concentration of 0.6 g/L. Hereinafter, the culture solution thus obtained is referred to as the culture solution D (additive).

Subsequently, the activated sludge in which the uronic acid unit concentration determined by the above-described method reached 100 mg/L was filtered with a filter paper 5C manufactured by Advantec MFS, Inc. to prepare a filtrate. 1 L of the filtrate was placed in a 5-L Erlenmeyer flask, 10 mL of the above-described culture solution D was aseptically added to the culture solution, and the thus obtained solution was subjected to a shaking and aeration treatment for 24 hours at a rate of 120 rpm while being maintained at 28°C.

The uronic acid unit concentration was measured with the above-described method 24 hours later and a value of 100 mg/L stayed about the same. The molecular weight distribution of the concerned solution was measured by applying a technique based on the same conditions as the conditions in Example 1 to verify that the peaks indicating the molecular weights of 1×10⁶ to 1×10⁸ Da did not change at all, and no degradation of the polysaccharides was verified.

### Comparative Example 2

An agar flat plate culture medium containing 0.15% of a phosphorus content, 0.04% of a nitrogen content and 0.001% of a Mg content, as well as 0.5 g/L of peptone (Sigma Aldrich Corp.) and 0.5 g/L of polygalacturonic acid (Sigma Aldrich Corp.) as carbon sources was prepared.

To the culture medium, an activated sludge, soil, and lake water were uniformly inoculated, and the culture medium was incubated at 28°C for 72 hours to form colonies of bacteria on the agar flat plate. The colony contained at least Nocardia sp., Castellaniella sp., Pseudomonas sp. and Humicoccus sp..

The colony of bacteria grown on the agar flat plate were aseptically inoculated with a platinum pool in 20 mL of a liquid culture medium that contains 0.15% of a phosphorus content, 0.04% of a nitrogen content and 0.001% of a Mg content as nutrient salts and further contains peptone in a concentration of 0.5 g/L and polygalacturonic acid in a concentration of 0.5 g/L.

The liquid culture medium was cultured for 24 hours while being maintained at 28°C, shaken at 120 rpm and aerated. Hereinafter, the culture solution thus obtained is referred to as the culture solution E (additive).

Subsequently, the activated sludge in which the uronic acid unit concentration determined by the above-described method reached 100 mg/L was filtered with a filter paper 5C manufactured by Advantec MFS, Inc. to prepare a filtrate. 1 L of the filtrate was placed in a 5-L Erlenmeyer flask, 10 mL of the above-described culture solution E was aseptically added to the culture solution, and the thus obtained solution was subjected to a shaking and aeration treatment for 24 hours at a rate of 120 rpm while being maintained at 28°C.

The uronic acid unit concentration was measured with the above-described method 24 hours later and a value of 100 mg/L stayed about the same. The molecular weight distribution of the concerned solution was measured by applying a technique based on the same conditions as the conditions in Example 1 to verify that the peaks indicating the molecular weights of 1×10⁶ to 1×10⁸ Da did not change at all, and no degradation of the polysaccharides was verified.

This application is based on the Japanese Patent Application No. 2009-231554, filed on October 5, 2009, which is herein incorporated entirely by reference.

### Industrial Applicability

The present invention is industrially applicable, since by adding the additive according to the present invention to the activated sludge, a polysaccharide is degraded which is a substance responsible for causing the membrane clogging in the membrane separation activated sludge method, and consequently the treatment of organic wastewater based on the membrane separation activated sludge method can be conducted stably over a long period of time. Additionally, the additive according to the present invention can be prepared by using an activated sludge, soil, lake water, river water and the like with an inexpensive, simple and easy method.

### Reference Signs List

- 1: Organic wastewater
- 2: Pretreatment equipment
- 3: Flow rate adjustment tank
- 4: Activated sludge tank (Aeration tank)
- 5: Separation membrane device
- 6: Skirt
- 7: Blower
- 8: Suction pump
- 9: Filtrate
- 10: Sterilization tank
- 11: Treated water
- 12: Sludge withdrawal pump

## Claims

1. An additive which is added to an activated sludge when organic wastewater is treated with a membrane separation activated sludge method, the additive comprising a microorganism to degrade a polysaccharide.

2. The additive according to claim 1, wherein the polysaccharide is a polysaccharide comprising an uronic acid unit.

3. The additive according to claim 1 or 2, wherein the polysaccharide is a polysaccharide comprising the uronic acid unit in a content of 5 to 70% based on total saccharide unit.

4. The additive according to any one of claims 1 to 3, wherein the polysaccharide is one or more polysaccharides selected from polygalacturonic acid, xanthan gum and hyaluronic acid.

5. The additive according to any one of claims 1 to 4, wherein the microorganism is one or more microorganisms selected from Penicillium sp., Phialemonium sp., Fluviicola sp., Pedobacter sp., Paenibacillus sp., Cohnella sp., Pseudoxanthomonas sp., Brevundimonas sp., Hydrogenophaga sp., Sphingomonas sp., Novosphingobium sp., Sphingopyxis sp., Microbacterium sp. Ochrobactrum sp., Sphingobacterium sp., Bacteroidetes bacterium, Xanthomonadaceae sp., Devosia sp., Prosthecomicrobium sp., Alpha proteobacterium sp. and Flexibacteraceae sp..

6. An organic wastewater-treating method based on a membrane separation activated sludge method comprising:
an inflow step in which an organic wastewater is made to flow in an activated sludge tank that contains a microorganism-containing activated sludge; and
a separation step in which a treated liquid obtained by biologically treating the organic wastewater in the activated sludge tank is subjected to a solid-liquid separation with a separation membrane device disposed in the activated sludge tank,
wherein an additive which contains a microorganism to degrade an uronic acid unit-containing polysaccharide is added to the activated sludge.

7. The organic wastewater-treating method according to claim 6, wherein the uronic acid unit concentration change over time in the water phase in the activated sludge tank is being measured, and the additive is added when the uronic acid unit concentration reaches 30 mg/L.

8. An organic wastewater-treating apparatus based on a membrane separation activated sludge method, comprising:
an activated sludge tank in which a microorganism-containing activated sludge is contained, and an organic wastewater is subjected to a biological treatment;
a separation membrane device disposed in the activated sludge tank or at a stage subsequent to the activated sludge tank and subjecting the biologically treated water to a solid-liquid separation;
a concentration-measuring means for measuring an uronic acid unit concentration change over time in the water phase in the activated sludge tank; and
an additive-adding means for adding in the activated sludge an additive that contains a microorganism to degrade an uronic acid unit-containing polysaccharide when the uronic acid unit concentration reaches a predetermined concentration.
